Europäisches Patentamt

European Patent Office

Office européen des brevets

(19)

(11) Publication number: **0 307 149 B1**

# EUROPEAN PATENT SPECIFICATION

(12)

(45) Date of publication of patent specification: **07.01.93**

(21) Application number: **88308170.5**

(22) Date of filing: **02.09.88**

(51) Int. Cl.⁵: **G01N 33/543**, G01N 33/569, C07K 13/00, C12N 15/00, C12P 21/02, A61K 39/21

(54) **Solid phase immunoassay for an antibody and biological constructions for use therein.**

(30) Priority: **04.09.87 GB 8720800**
**28.07.88 GB 8818030**

(43) Date of publication of application:
**15.03.89 Bulletin 89/11**

(45) Publication of the grant of the patent:
**07.01.93 Bulletin 93/01**

(84) Designated Contracting States:
**AT BE CH DE ES FR GB GR IT LI LU NL SE**

(56) References cited:
**EP-A- 0 139 216          EP-A- 0 152 030**
**EP-A- 0 187 041          EP-A- 0 214 555**
**WO-A-86/04423          WO-A-86/06414**

**CURRENT TOPICS IN AIDS, vol. 1, 1987, John Wiley & Sons Ltd.; M.S.GOTTLIEB et al., pp. 95-102**

**VIROLOGY, vol. 136, no. 2, July 30, 1984; S.HATTORI et al., pp. 338-347**

(73) Proprietor: **INTERNATIONAL MUREX TECH-NOLOGIES CORPORATION**
**Suite 600 70 University Avenue**
**Toronto, CANADA(CA)**

(72) Inventor: **Highfield, Peter Edmund**
**The Wellcome Foundation Limited, Langley Court**
**Beckenham,Kent, BR3 3BS(GB)**
Inventor: **Duncan, Richard Julian Stuart**
**The Wellcome Foundation Limited Langley Court**
**Beckenham Kent, BR3 3BS(GB)**
Inventor: **Parker, David**
**The Wellcome Foundation Limited Langley Court**
**Beckenham Kent, BR3 3BS(GB)**
Inventor: **Spence, Robert Paul**
**The Wellcome Foundation Limited Langley Court**
**Beckenham Kent, BR3 3BS(GB)**

(74) Representative: **Silveston, Judith et al**
**ABEL & IMRAY Northumberland House**
**303-306 High Holborn**
**London, WC1V 7LH(GB)**

Rank Xerox (UK) Business Services

EP 0 307 149 B1

## Description

This invention relates to immunoassays for antibodies, to a protein and to test kits for use in such immunoassays. It further relates to a pharmaceutical composition containing the protein.

The most common expression systems for cloning antigens are those in which the antigens are expressed in E. Coli However, E. Coli occurs noticeably as part of the gut flora. Many human sera therefore contain antibodies to E. Coli. When antigens produced in E. Coli are used in sandwich immunoassays for antibody, there is the possibility that some individuals may react with contaminating bacterial Material. Such a reaction may give a false positive result for that particular individual. This type of false positive may be minimised by mixing material from E. Coli with a test sample.

We have now devised a new way of overcoming the problem of contaminating material. In essence, immunologically identical antigens are engineered into different organisms. Cloned antigen from different organisms is therefore provided on either side of the "sandwich". Antibody it is not wished to detect in a test sample may bind to contaminating material associated with one of the cloned antigens but, because they are from different sources, not to contaminants associated with both cloned antigens. False positives can thus be avoided. Importantly, it is not then necessary to ensure that the cloned antigens are absolutely free of contaminating proteins. The specificity of the assay is greatly improved. The preparation of the antigens is simplified.

Accordingly, the present invention provides an immunoassay for an antibody, which immunoassay comprises:

(i) contacting a solid phase, on which is immobilised a first recombinant peptide which presents an antigenic sequence to which the antibody is capable of binding, with a test sample;

(ii) contacting the solid phase with a second recombinant peptide which presents the said antigenic sequence, which is labelled and which was expressed in an organism of a different genus than that in which the first recombinant peptide was expressed; and

(iii) determining whether the test sample contained any said antibody.

Any antibody may be assayed for in this way. The recombinant peptides must both include the same antibody binding site. Detection depends on the fact that antibodies have at least two antigen combining sites, two for IgG, IgA and IgE and five for IgM. Taking IgG as an example, one of the antigen combining sites binds with the first recombinant peptide which is immobilised on a solid phase. The labelled second recombinant peptide binds with the second antigen combining site of IgG.

The recombinant peptides are engineered in organisms of a different genus. Preferably, each organism is of a different family. For example, different bacterial expression systems may be used. Alternatively, one antigen cloned in a bacterial expression system and one cloned in yeast or in insect or mammalian cells may be used. Most preferably, one antigen is cloned in a procaryotic organism whilst the other is cloned in a eucaryotic system. Examples of suitable hosts for cloning include B. subtilis, E. Coli, Streptomyces, insect cells, yeast and mammalian cells. A baculovirus expression system or a vaccinia virus expression system, in which peptides are expressed in insect and mammalian cells respectively, are preferred eucaryotic alternatives. E. Coli is a preferred procaryotic host.

The first and second recombinant peptides need not be identical in structure. It is permissible for one to be longer than the other. They must both present the antigenic sequence to which the antibody it is wished to detect is capable of binding, however. In other words, each must include the same antibody binding site. By the term "peptide" we include proteins.

Any appropriate label may be attached to the second recombinant peptide. The label may be an enzyme, radioisotope or other reagent which provides colorimetric or fluorometric activity. A preferred label is alkaline phosphatase. A cyclic amplification reaction can be initiated using the label. The alkaline phosphatase reacts with the substrate nicotinamide adenine dinucleotide phosphate (NADP) to form nicotinamide adenine dinucleotide (NAD). The NAD is utilised in a cycling reaction involving alcohol dehydrogenase, diaphorase and iodonitrotetrazolium violet which leads to a coloured product.

The immunoassay is particularly suitable for use in determining the presence of HIV antibody, especially HIV-1 antibody, in a sample. Cloned HIV antigens from different sources may be provided. The antigens may be gag and/or env sequences. HIV-1 provokes in particular two types of antibody. These are anti-p24 against the gag protein and anti-gp41 against the env protein.

Our preferred gag sequence corresponds to amino acids 121-356. This incorporates all of the sequence of p24. Our preferred env sequence corresponds to amino acids 542-674. Advantages of this gp41 sequence are that it is semi-soluble and that it includes the dominant epitope of the env gene. The numbering is according to Meusing et al Nature 313 450-458 (1985).

Preferably, each sequence is fused to β-galactosidase (β-gal). This facilitates purification by affinity

2

chromatography, using either an anti-galactosidase affinity column or a substrate column. Alternatively, the two sequences may be fused together as a gag/env fusion protein. Early seroconversion may be against either the gag or the env antigen or both. Desirably, therefore, both the first and the second recombinant peptides present both the gag and the env sequences. The gag/β-gal and env/β-gal fusion proteins may be used to achieve this or the gag/env fusion protein may be used on its own.

A preferred gag/env fusion protein is a protein of the sequence:

```
                              10                              20
MetAsnSerProAspThrGlyHisSerSerGlnValSerGlnAsnTyrProIleValGln
        p18>                                        p24>

                              30                              40
AsnIleGlnGlyGlnMetValHisGlnAlaIleSerProArgThrLeuAsnAlaTrpVal

                              50                              60
LysValValGluGluLysAlaPheSerProGluValIleProMetPheSerAlaLeuSer

                              70                              80
GluGlyAlaThrProGlnAspLeuAsnThrMetLeuAsnThrValGlyGlyHisGlnAla

                              90                             100
AlaMetGlnMetLeuLysGluThrIleAsnGluGluAlaAlaGluTrpAspArgValHis

                             110                             120
ProValHisAlaGlyProIleAlaProGlyGlnMetArgGluProArgGlySerAspIle

                             130                             140
AlaGlyThrThrSerThrLeuGlnGluGlnIleGlyTrpMetThrAsnAsnProProIle

                             150                             160
ProValGlyGluIleTyrLysArgTrpIleIleLeuGlyLeuAsnLysIleValArgMet

                             170                             180
TyrSerProThrSerIleLeuAspIleArgGlnGlyProLysGluProPheArgAspTyr

                             190                             200
ValAspArgPheTyrLysThrLeuArgAlaGluGlnAlaSerGlnGluValLysAsnTrp

                             210                             220
MetThrGluThrLeuLeuValGlnAsnAlaAsnProAspCysLysThrIleLeuLysAla

                             230                             240
LeuGlyProAlaAlaThrLeuGluGluMetMetThrAlaCysGlnGlyValGlyGlyPro

                             250                             260
AsnSerProArgGlnLeuLeuSerGlyIleValGlnGlnGlnAsnAsnLeuLeuArgAla
        gp41>

                             270                             280
IleGluAlaGlnGlnHisLeuLeuGlnLeuThrValTrpGlyIleLysGlnLeuGlnAla

                             290                             300
ArgIleLeuAlaValGluArgTyrLeuLysAspGlnGlnLeuLeuGlyIleTrpGlyCys

                             310                             320
SerGlyLysLeuIleCysThrThrAlaValProTrpAsnAlaSerTrpSerAsnLysSer
```

3

```
                     330                                    340
LeuGluGlnIleTrpAsnAsnMetThrTrpMetGluTrpAspArgGluIleAsnAsnTyr

                     350                                    360
ThrSerLeuIleHisSerLeuIleGluGluSerGlnAsnGlnGlnGluLysAsnGluGln

                     370
GluLeuLeuGluLeuAspLysTrpAlaSerLeuTrpAsnTrpPheAsnGlyAspPro;
```

optionally modified by one or more amino acid substitutions, insertions and/or deletions and/or by an extension at either or both ends provided that a protein having such a modified sequence is capable of binding to both anti-p24 and anti-gp41 and there is a degree of homology of at least 75% between the modified and the unmodified sequences.

The unmodified sequence is basically a fusion of parts of the p24 and gp41 proteins of the CBL-1 isolate of HIV-1 (WO 86/04423). These parts correspond to amino acids 121 to 356 and 542 to 674 respectively. The start of these parts is shown above at amino acids 17 and 244 respectively. Amino acids 5 to 16 above are derived from the p18 protein. Amino acids 1 to 4, 241 to 243 and 377 to 379 above are derived from the expression vector from which the fusion construct was obtained and from DNA manipulations.

The sequence may be modified by one or more amino acid substitutions, insertions and/or deletions. These may occur anywhere in the sequence but especially in the parts of the sequence which are not derived from the p24 and gp41 proteins. In the case of substitutions, one or more of the amino acids of the unmodified sequence may be substituted by one or more other amino acid which preserves the physicochemical character of the sequence, i.e. in terms of charge density, hydrophilicity/hydrophobicity, size and configuration. For example, Ser may be replaced by Thr and vice versa, Glu may be replaced by Asp and vice versa and Gln may be replaced by Asn and vice versa. The Ser residue at amino acid 10 may be replaced by Asn.

The sequence may also be extended on one or both ends. This may be no more than the provision of an additional carboxy-terminal Cys residue. However, the sequence may be extended by up to 50 amino acid residues at either or both ends. Up to 40 amino acids, for example up to 20 amino acids, may therefore be added to the amino-terminus and/or carboxy-terminus of the unmodified sequence. The amino-terminal amino acid, however, will normally be Met due to the translational start codon of the nucleic acid sequence from which the protein is expressed. This is unless the protein has been expressed fused at its amino-terminus to a carrier protein and the fusion protein has been cleaved to release the protein of the invention.

The sequence may be modified by introducing corresponding changes into the DNA sequence encoding the unmodified protein. This may be achieved by any appropriate technique, including restriction of the sequence with an endonuclease, insertion of linkers, use of an exonuclease and/or a polymerase and site-directed mutagenesis techniques. Whether the modified DNA sequence encodes a modified protein to which both anti-p24 and anti-gp41 are capable of binding can be readily determined. The modified sequence is cloned into an appropriate plasmid, a host cell is transformed with the plasmid and the protein that is expressed is tested for its ability to bind anti-p24 and anti-gp41. Also, there must be a degree of homology of at least 75%, for example of 85% or more or of 90% or more, between the amino acid sequences of the modified and unmodified proteins.

The fusion proteins gag/β-gal, env/β-gal and gag/env can be expressed in E. Coli transformants. Such gag/β-gal and env/β-gal fusion proteins or such a gag/env fusion protein can be on one side of a "sandwich". Preferably, it is these fusion proteins which are labelled and which constitute the second recombinant peptide(s).

As the source of the gag and env antigens on the other side of the "sandwich", any appropriate expression system may be used. Preferably, however, a baculovirus expression system is used in which the polyhedrin gene incorporates or is replaced by a DNA sequence encoding the antigenic gag and/or env sequences. The recombinant peptide is expressed in insect cells. Typically, the host is Spodoptera frugiperda. A larger env sequence may be expressed which incorporates at least the amino acids 542-674, for example an env sequence corresponding to amino acids 24-750. The peptides cloned using the baculovirus expression system are preferably the peptides which are immobilised on the solid phase.

In carrying out the immunoassay, the first recombinant peptide is immobilised on a solid phase. The solid phase may be polystyrene beads, plastic microwells, etc. The peptide may be adsorbed onto the solid phase or bound there by antibody. A test sample is then brought into contact with the solid phase. A sample of any appropriate physiological fluid may constitute the test sample, for example urine, plasma or serum. Antibody in the test sample which is specific for the peptide immobilised on the solid phase binds to the peptide. Any antibody in the test sample which is specific for contaminating material derived from the host in which the peptide was cloned may bind to such material.

The labelled second recombinant peptide is also brought into contact with the solid phase. This may be done after the test sample has been brought into contact with the solid phase. Alternatively, the two steps can be effected simultaneously. Antibody in the test sample which binds to the first peptide also binds to the labelled second peptide and can thus be detected. Antibody in the test sample which binds to the host-derived contaminants associated with the first peptide does not bind to any labelled host-derived contaminants associated with the second peptide and vice versa. False positives may thus be avoided.

The immunoassay can be effected qualitatively, i.e. it can be carried out simply to detect the presence or absence of a particular antibody in a test sample. Alternatively, it may be effected quantitatively or semi-quantitatively to give a measure of how much antibody there is in the sample.

The materials for use in the immunoassay may be presented in a test kit. Such a test kit typically comprises:

(a) a solid phase on which is immobilised a first recombinant peptide which presents an antigenic sequence to which the antibody is capable of binding; and

(b) a second recombinant peptide which presents the said antigenic sequence, which is labelled and which was expressed in an organism of a different genus than that in which the first recombinant peptide was expressed.

The kit may also include a substrate for the label where the label is an enzyme. Further components can be wash fluids and controls. The kit may also contain materials for carrying out a second immunoassay alongside the present immunoassay. The second immunoassay may be for a different antibody, in which case it may be effected in a similar fashion.

If the second immunoassay is for the purpose of determining whether a test sample also contains a particular antigen, immobilised on the solid phase may be additionally a first antibody capable of binding to the antigen. A labelled second antibody for detecting antigen bound to the first antibody is also provided. Each antibody may be monoclonal or polyclonal. The two antibodies may be capable of binding to different sites on the antigen. Where the antigen is polymeric, the antibodies can be capable of binding to the same site. The labelled second antibody is brought into contact with the solid phase at the same time as the test sample or subsequently. The label may be the same as that on the second recombinant peptide when all that is required is an indication whether the test sample contains the antibody and/or antigen being tested for.

It is thus possible to provide an immunoassay for both HIV antibody, in particular HIV-1 antibody, and hepatitis B surface antigen (HBsAg). Assay for HIV antibody may be effected using the antigens already described. Two different antibodies for HBsAg can be employed to assay for the HBsAg, each reacting with a different site on the antigen. Each antibody may be monoclonal or polyclonal. Preferably, each is a monoclonal antibody so that the solid phase may be contacted with the test sample and the labelled second antibody simultaneously. Again preferably, the labels on the second recombinant peptide for use in detecting the HIV antibody and on the second HBsAg-specific antibody are the same.

The immunoassay for HIV antibody may be used as a prognostic test. HIV-1 infected subjects initially have a fairly constant high titre of anti-p24 antibody when free of AIDS symptoms. On the other hand, a lower or falling titre of anti-p24 antibody is significantly associated with clinical progression and provides an indicator for the onset of AIDS or AIDS-related complex (ARC) (Weber et al (1987) The Lancet, 17 January).

In order to effect a prognostic test, it is necessary therefore to follow the anti-p24 antibody titre in a subject over a period of time. An immunoassay may therefore be conducted using cloned p24 peptides obtained from different organisms. Preferred are the p24 constructs discussed above. When the anti-p24 antibody titre drops, this is considered to be a marker for a poor prognosis.

The present invention also relates to fusion constructs, their preparation and their use in assaying for anti-HIV-1 antibody and as a vaccine.

A variety of assays have been proposed for anti-HIV-1 antibody. However, there are problems with false positive and false negative results arising. HIV-1 provokes in particular two types of antibody. These are anti-p24 against the gag protein and anti-gp41 against the env protein. We have now prepared a specific fusion construct to which both anti-p24 and anti-gp41 bind. This enables accurate and sensitive assays to be carried out without the risk of false positive or false negative results.

Accordingly, the present invention provides a protein of the sequence:

```
                              10                        20
MetAsnSerProAspThrGlyHisSerSerGlnValSerGlnAsnTyrProIleValGln
          p18>                              p24>

                              30                        40
AsnIleGlnGlyGlnMetValHisGlnAlaIleSerProArgThrLeuAsnAlaTrpVal

                              50                        60
```

LysValValGluGluLysAlaPheSerProGluValIleProMetPheSerAlaLeuSer

70                                                    80
GluGlyAlaThrProGlnAspLeuAsnThrMetLeuAsnThrValGlyGlyHisGlnAla

90                                                    100
AlaMetGlnMetLeuLysGluThrIleAsnGluGluAlaAlaGluTrpAspArgValHis

110                                                   120
ProValHisAlaGlyProIleAlaProGlyGlnMetArgGluProArgGlySerAspIle

130                                                   140
AlaGlyThrThrSerThrLeuGlnGluGlnIleGlyTrpMetThrAsnAsnProProIle

150                                                   160
ProValGlyGluIleTyrLysArgTrpIleIleLeuGlyLeuAsnLysIleValArgMet

170                                                   180
TyrSerProThrSerIleLeuAspIleArgGlnGlyProLysGluProPheArgAspTyr

190                                                   200
ValAspArgPheTyrLysThrLeuArgAlaGluGlnAlaSerGlnGluValLysAsnTrp

210                                                   220
MetThrGluThrLeuLeuValGlnAsnAlaAsnProAspCysLysThrIleLeuLysAla

230                                                   240
LeuGlyProAlaAlaThrLeuGluGluMetMetThrAlaCysGlnGlyValGlyGlyPro

250                                                   260
AsnSerProArgGlnLeuLeuSerGlyIleValGlnGlnGlnAsnAsnLeuLeuArgAla
        gp41>

270                                                   280
IleGluAlaGlnGlnHisLeuLeuGlnLeuThrValTrpGlyIleLysGlnLeuGlnAla

290                                                   300
ArgIleLeuAlaValGluArgTyrLeuLysAspGlnGlnLeuLeuGlyIleTrpGlyCys

310                                                   320
SerGlyLysLeuIleCysThrThrAlaValProTrpAsnAlaSerTrpSerAsnLysSer

330                                                   340
LeuGluGlnIleTrpAsnAsnMetThrTrpMetGluTrpAspArgGluIleAsnAsnTyr

350                                                   360
ThrSerLeuIleHisSerLeuIleGluGluSerGlnAsnGlnGlnGluLysAsnGluGln

370
GluLeuLeuGluLeuAspLysTrpAlaSerLeuTrpAsnTrpPheAsnGlyAspPro;

optionally modified by one or more amino acid substitutions, insertions and/or deletions and/or by an extension at either or both ends provided that a protein having such a modified sequence is capable of binding to both anti-p24 and anti-gp41 and there is a degree of homology of at least 75% between the modified and the unmodified sequences.

The unmodified sequence is basically a fusion of parts of the p24 and gp41 proteins of the CBL-1 isolate of HIV-1 (WO 86/04423). These parts correspond to amino acids 121 to 356 and 542 to 674 respectively, following a similar numbering system to that of Meusing et al, Nature, 313, 450-458 (1985). The start of these parts is shown above at amino acids 17 and 244 respectively. Amino acids 5 to 16 above are derived from the p18 protein. Amino acids 1 to 4, 241 to 243 and 377 to 379 above are derived from the expression vector from which the fusion construct was obtained and from DNA manipulations.

The sequence may be modified by one or more amino acid substitutions, insertions and/or deletions. These may occur anywhere in the sequence but especially in the parts of the sequence which are not derived from the p24 and gp41 proteins. In the case of substitutions, one or more of the amino acids of the unmodified sequence may be substituted by one or more other amino acid which preserves the physicochemical character of the sequence, i.e. in terms of charge density, hydrophilicity/hydrophobicity, size and configuration. For example, Ser may be replaced by Thr and vice versa, Glu may be replaced by Asp and vice versa and Gln may be replaced by Asn and vice versa. The Ser residue at amino acid 10 may be replaced by Asn.

The sequence may also be extended on one or both ends. This may be no more than the provision of an additional carboxy-terminal Cys residue. However, the sequence may be extended by up to 50 amino acid residues at either or both ends. Up to 40 amino acids, for example up to 20 amino acids, may therefore be added to the amino-terminus and/or carboxy-terminus of the unmodified sequence. The amino-terminal amino acid, however, will normally be Met due to the translational start codon of the nucleic acid sequence from which the protein is expressed. This is unless the protein has been expressed fused at its amino-terminus to a carrier protein and the fusion protein has been cleaved to release the protein of the invention.

The sequence may be modified by introducing corresponding changes into the DNA sequence encoding the unmodified protein. This may be achieved by any appropriate technique, including restriction of the sequence with an endonuclease, insertion of linkers, use of an exonuclease and/or a polymerase and site-directed mutagenesis techniques. Whether the modified DNA sequence encodes a modified protein to which both anti-p24 and anti-gp41 are capable of binding can be readily determined. The modified sequence is cloned into an appropriate plasmid, a host cell is transformed with the plasmid and the protein that is expressed is tested for its ability to bind anti-p24 and anti-gp41. Also, there must be a degree of homology of at least 75%, for example of 85% or more or of 90% or more, between the amino acid sequences of the modified and unmodified proteins.

A protein of the invention is prepared by a process comprising:
(i) transforming a host cell with a vector which incorporates a gene encoding the protein and which is capable, in the host cell, of expressing the protein;
(ii) culturing the transformed host cell so that the protein is expressed; and
(iii) recovering the protein.

The gene encoding the protein is preferably constructed in two parts, an amino-terminal part incorporating the DNA sequence encoding the p24 amino acid residues and a carboxy-terminal part incorporating the DNA sequence encoding the gp41 amino acid residues. The two parts are then fused together and inserted in an expression vector. The gene is provided with appropriate transcriptional regulatory sequences and translational start and stop codons. The gene is therefore inserted in the expression vector in the correct reading frame with respect to an ATG start codon under the control of a promoter.

Any suitable expression vector may be employed, for example a plasmid or a viral vector such as a recombinant baculovirus or recombinant vaccinia virus. The host transformed with the expression vector may be eucaryotic or procaryotic, for example unicellular microorganisms or mammalian cells. As a unicellular eucaryotic host, Saccharomyces cerevisiae, S. kluveromyces and S. pombe may be mentioned. Strains of bacteria such as E. coli, B. subtilis or B. thermophilus may be used as procaryotic hosts. E. coli is preferred. The transformed host is cultured and the protein that is expressed is recovered.

A protein of the invention can be used in assays for anti-HIV-1 antibody, in particular for anti-p24 and/or anti-gp41. A test sample of any appropriate physiological fluid may be used in the assay, for example urine, plasma, blood or serum. The assay method comprises contacting a test sample with a protein of the invention and determining whether any anti-p24 and/or anti-gp41 binds to the protein. For this purpose, a test kit may be provided comprising a protein of the invention and means for determining whether any anti-p24 and/or anti-gp41 there may be in a test sample binds to the protein.

A variety of assay formats may be employed. The protein can be used to selectively capture anti-p24 and/or anti-gp41 from solution, to selectively label anti-p24 and/or anti-gp41 already captured, or to both capture and label. In addition the protein may be used in a variety of homogeneous assay formats in which the antibodies which react with the protein are detected in solution with no separation of phases. The protein can also be used for HIV-1 antigen detection.

The types of assay in which the protein is used to capture antibodies from solution involve immobilization of the protein onto a solid surface. This surface should be capable of being washed in some way. The sort of surfaces which may be used are polymers of various types (moulded into microtitre wells; beads; dipsticks of various types; aspiration tips; electrodes; and optical devices), particles (for example latex;

stabilized blood, bacterial or fungal cells; spores; gold or other metallic sols; and proteinaceous colloids; with the usual size of the particle being from 0.1 to 5 microns), membranes (for example nitrocellulose; paper; cellulose acetate; and high porosity/high surface area membranes of an organic or inorganic material).

The attachment of the protein to the surfaces can be by passive adsorption (for which it is ideally suited by virtue of its hydrophobic nature) from a solution of optimum composition which may include surfactants, solvents, salts, chaotropes; or by active chemical bonding. Active bonding may be through a variety of reactive or activatible functional groups which may be attached to the surface (for example condensing agents; active esters, halides, anhydrides; amino, hydroxyl, or carboxyl groups; sulphydryl groups; carbonyl groups; diazo groups; unsaturated groups). Alternatively the active bonding may be through another protein (itself attached to the surface passively or through active bonding), e.g. through polyclonal or monoclonal antibody directed against any or all of the epitopes presented by the protein, or through a carrier protein such as albumin or casein, to which the protein may be chemically bonded by any of a variety of methods and which may confer advantages because of isoelectric point, charge, hydrophilicity or other physico-chemical property. The protein may also be attached to the surface (usually but not necessarily a membrane) following electrophoretic separation of a reaction mixture e.g. an immune precipitation.

After reacting the surface bearing the protein with a solution contain antibody of interest and removing the excess of the sample where necessary by any of a variety of means (washing, centrifugation, filtration, magnetism, capilliary action), the captured antibody is detected by any means which will give a detectable signal. For example, this may be achieved by use of a labelled molecule or particle as defined above which will react with the captured antibody (for example protein A or protein G and the like; anti-species or anti-immunoglobulin-sub-type; rheumatoid factor; antibodies to any of the epitopes contained in the protein and used in a competitive or blocking fashion; or any molecule containing the epitopes of the protein, including the protein itself and other proteins and peptides derived directly or indirectly from HIV-1).

The detectable signal may be optical or radio-active or physico-chemical, provided by directly labelling the molecule referred to with for example a dye, radiolabel, electroactive species, magnetically resonant species or fluorophore; or indirectly by labelling the molecule or particle with an enzyme itself capable of giving rise to a measurable change of any sort. Alternatively the detectable signal may be due to, for example, agglutination, diffraction effect or birefringent effect occurring if any of the surfaces referred to are particles.

Those types of assay in which the protein is used to label an already captured antibody require some form of labelling of the protein which will allow it to be detected. The labelling can be direct, by chemically or passively attaching for example a radio-, magnetic resonant-, particle or enzyme label to the protein; or indirect by attaching any form of label to a molecule which will itself react with the protein, e.g. antibody to any of the epitopes of the protein, with subsequent reaction of the labelled molcule with the protein. The chemistry of bonding a label to the protein can be directly through a moiety already present in the protein, such as an amino or sulphydryl or through an inserted group such as a maleimide. Capture of the antibody may be on any of the surfaces already mentioned, by any reagent, including passive or activated adsorption, which will result in specific antibody or immune complexes being bound. In particular capture of the antibody could be by anti-species or anti-immunoglobulin-sub-type, by rheumatoid factor, proteins A, G and the like, or by any molecule containing any of the epitopes of the protein as described above.

For those assays in which the protein is used to provide a measure of HIV-1 antigen in a sample, the protein may be labelled in any of the ways described above, and used in either a competitive binding fashion so that its binding by any specific molecule on any of the surfaces exemplified above is blocked by antigen in the sample, or in a non-competitive fashion when antigen in the sample is bound specifically or non-specifically to any of the surfaces above, in turn binds a specific bi- or poly-valent molecule (e.g. an antibody) and the remaining valances of the molecule are used to capture the labelled protein.

In general in homogeneous assays the protein and an antibody are labelled, so that, when the antibody reacts with the protein in free solution, the two labels interact, for example to allow non-radiative transfer of energy captured by one label to the other label, with appropriate detection of the excited second label or quenched first label (e.g. by fluorimetry, magnetic resonance or enzyme measurement). Addition of either antigen or antibody in a sample results in restriction of the interaction of the labelled pair, and so to a different level of signal in the detector.

The protein of the invention may be used in a sandwich immunoassay for anti-p24 or anti-gp41 in which recombinant antigens, which present the same antigenic sequence but which have been expressed in different organisms, are present on either side of the sandwich. The most common expression systems for cloning antigens are those in which the antigens are expressed in E. coli. However, E. coli occurs noticeably as part of the gut flora. Many human sera therefore contain antibodies to E. coli. When antigens produced in E. coli are used in sandwich immunoassays for antibody, there is the possibility that some individuals may react with contaminating bacterial material. Such a reaction may give a false positive result for that particular individual. This type of false positive may be minimised by mixing material from E. coli with a test sample.

We have now devised a new way of overcoming the problem of false positives due to antibodies to contaminating material. In essence, immunologically identical antigens are engineered in different organisms. Cloned antigen from different organisms is therefore provided on either side of the "sandwich". Antibody it is not wished to detect in a test sample may bind to contaminating material associated with one of the cloned antigens but, because they are from different sources, not to contaminants associated with both cloned antigens. False positives can thus be avoided. Importantly, it is not then necessary to ensure that the cloned antigens are absolutely free of contaminating proteins. The specificity of the assay is greatly improved. The preparation of the antigens is simplified.

In essence, an immunoassay in this format for anti-p24 and/or anti-gp41 antibody comprises:

(i) contacting a solid phase, on which is immobilised a first recombinant peptide which presents an antigenic sequence to which the antibody is capable of binding, with a test sample;

(ii) contacting the solid phase with a second recombinant peptide which presents the said antigenic sequence, which is labelled and which was expressed in a different organism than the first recombinant peptide; and

(iii) determining whether the test sample contained any said antibody.

The first recombinant peptide and/or the second recombinant peptide is a recombinant protein according to the invention. The first and second recombinant peptides need not be identical in structure. It is permissible for one to be longer than the other, for example. They must both present the antigenic sequence to which the anti-p24 and/or anti-gp41 it is wished to detect are/is capable of binding, however. In other words, each must include the same antibody binding site(s).

Two recombinant proteins of different sequences according to the invention or two recombinant proteins of the same sequence according to the invention may therefore by used. Alternatively, only one of the recombinant peptides is a protein according to the invention. In these circumstances, however, the other peptide and the recombinant protein according to the invention must present a common epitope. The other recombinant peptide may be a gag/env fusion or just a gag or an env peptide.

Detection of anti-p24 and/or anti-gp41 depends on the fact that antibodies have at least two antigen combining sites, two for IgG, IgA and IgE and five for IgM. Taking IgG as an example, one of the antigen combining sites binds with the first recombinant peptide which is immobilised on a solid phase. The labelled second recombinant peptide binds with the second antigen combining site of IgG.

The recombinant peptides in this assay format are engineered in different organisms. Preferably, the genus at least of each organism is different. More preferably, each organism is of a different family. For example, different bacterial expression systems may be used. Alternatively, one antigen cloned in a bacterial expression system and one cloned in yeast or in insect or mammalian cells may be used. Most preferably, one antigen is cloned in a procaryotic organism whilst the other is cloned in a eucaryotic system. Examples of suitable hosts for cloning include B. subtilis, E. coli, Streptomyces, insect cells, yeast and mammalian cells. A baculovirus expression system or a vaccinia virus expression system, in which peptides are expressed in insect and mammalian cells respectively, are preferred eucaryotic alternatives. E. coli is a preferred procaryotic host.

A protein of the invention may also be used as a vaccine against HIV-1. For this purpose, the protein may be formulated in a pharmaceutical composition with a pharmaceutically acceptable carrier or diluent. The protein may be presented as an injectable formulation. Suitable diluents include water for injections and isotonic saline solution. The composition is administered parenterally, for example intravenously, intramuscularly or subcutaneously. An effective amount is given to a human. Typically a dose of from 10 to 200ug is given parenterally.

The following Examples illustrate the invention.

In the accompanying drawings:

Figure 1 shows the preferred env sequence as incorporated in pDM322 in Example 1;

Figure 2 shows the preferred gag sequence as incorporated in pDM614 in Example 1;

Figure 3 shows the DNA sequence of the gag/env protein of Example 1 with vector-related sequences are shown in bold;

Figure 4 shows the construction of pFOHc in Example 3;

Figure 5 shows the shuttle vector pvFOHC, the sequence of which contains two in-phase initiation codons separated by the FMDV VP1 142-160 sequence and six amino acids of the authentic HB "pre-core" sequence;

Figure 6 presents the results of the sandwich ELISA in Example 3; and
Figure 7 shows the sucrose gradient profile of core reactive material obtained in Example 3.

Example 1. E. coli expression constructions

A lambda gt10 (DNA Cloning vol. 1, Editor - D.M. Glover, IRL Press 1985) cDNA library was constructed from poly(A+)-RNA from CEM cells infected with the British isolate of HIV (CBL-1) by standard techniques (Molecular Cloning (1982) Maniatis et al Cold Spring Harbor Press). Cells of a leukaemic T-cell line designated CCRF-CEM which harbour CBL-1 have been deposited at ECACC, Porton Down, GB on 11 January 1985 under deposit number 85 01 1101.

A lambda recombinant containing the entire envelope gene was identified and the EcoRI insert fragment was subcloned into the plasmid vector pUC8 to produce the plasmid pDP4. The exact location of the envelope gene within pDP4 was determined by comparison with published data following restriction enzyme analysis and partial DNA sequencing (Meusing et al (1985) Nature 313 450-458, Wain-Hobson et al (1985) Cell 40 9-17, Sanchez-Pescador et al (1985) Science 227 484-492).

We expressed parts of the HIV genome in E. coli by fusing fragments to the lacZ gene. The expression vector chosen was pXY460. This is an Open Reading Frame vector where the strong tac promoter drives a mutated lacZ gene in which the initiating ATG and the coding sequence for $\beta$-galactosidase are out of frame. The vector is derived from pXY410 (Winther et al J. Immunol. 136 (1986) 1835-1840). There are restriction sites for EcoRI, Smal and BamHI at the start of the lacZ gene. Any DNA fragments inserted here which can restore the reading frame will produce fusion proteins consisting of the sequence encoded by the insert attached to $\beta$-galactosidase.

```
            EcoRI              BamHI
    ... ATG AAT TCC C GGG GAT CC. ... ... lacZ
                   SmaI
```

Vector pXY460, harboured in E. coli HB101, was deposited at NCIMB, Aberdeen, GB on 26 July 1988 under accession number NCIB 40039.

a) Envelope

The region of the HIV-1 env gene coding for amino acids 542-674 was cloned into pXY460 in the following manner. A DNA sequence containing a BamHI restriction site was introduced into the gene at nucleotide position 8276. The result of this was to add on two amino acids (Gly-Asp) at position 674 and to introduce a BamHI site in-frame with the pXY460 vector.

```
              674       BamHI
    env....TGG TTT AAC GGG GAT CC. ... ...
       ...Trp Phe Asn Gly Asp Pro ...
```

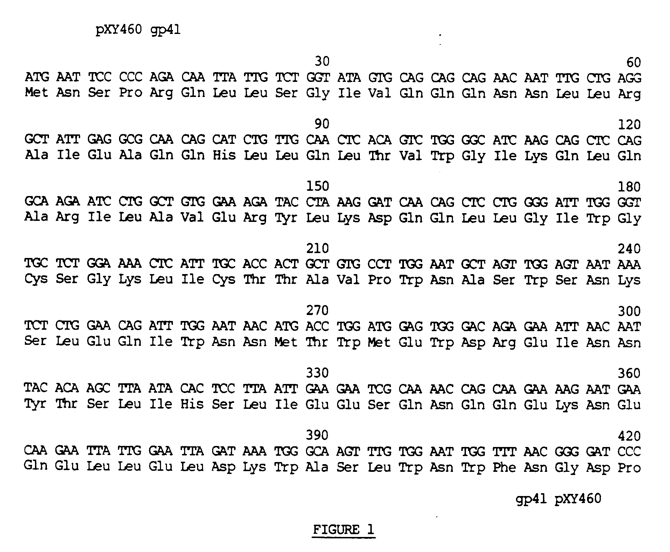

There is a site for the enzyme HaeIII at position 7875 (amino acid 541) which is in-frame with the Smal site in pXY460. The 403bp HaeIII/BamHI fragment was purified by elution from an agarose gel slice and ligated with Smal/BamHI digested pXY460. The ligated DNA was transformed into E. coli strain TG1 and recombinants were selected as blue colonies on L-Amp-Xgal agar plates. Individual transformants were characterised by restriction enzyme digestion and one of those with the predicted pattern of fragments was called pDM322. The env sequence incorporated in pDM322 is shown in Figure 1.

b) Gag (core)

The region of the HIV-1 gag gene which encodes amino acids 121-356 was cloned into pXY460 in the following manner.

There is a site for the enzyme NciI [CC(C/G)GG] at position 1180 (amino acid 356) within the gag gene.

```
                        NciI
        ... GGA GGA CCC GGC ... ...
            Gly Gly Pro Gly
```

The 5′ overhang generated by this enzyme was filled in by incubation with the Klenow fragment of E. coli DNA polymerase I in the presence of only dCTP. This end was then in-frame with the SmaI site in pXY460. Similarly the PvuII site at position 468 (amino acid 121) gave a blunt end in-frame with the SmaI site in pXY460.

```
                        PvuII
        ... GCA G CA GCT GAC ... ...
            Ala A la Ala Asp
```

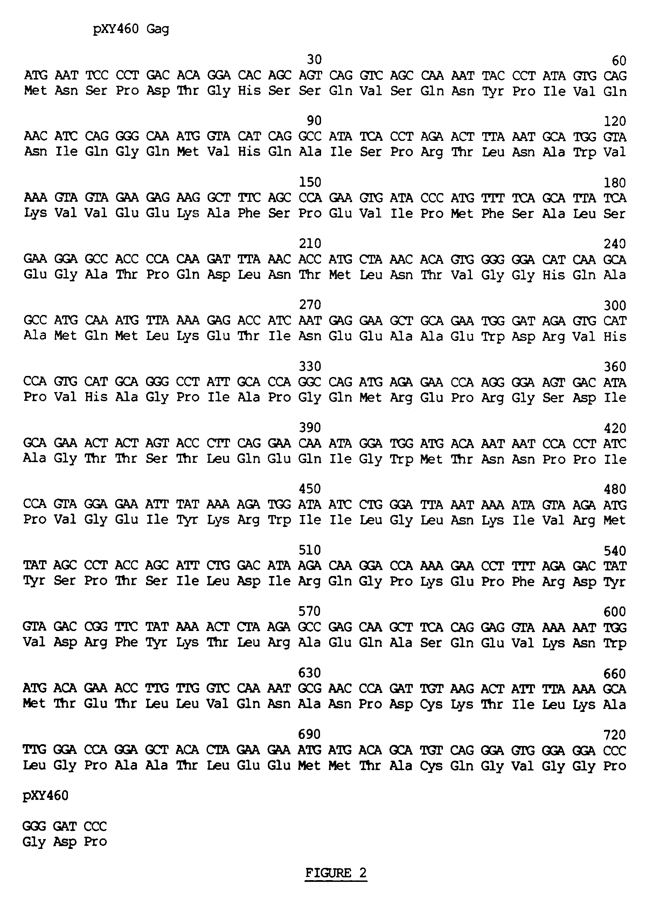

The PvuII/NciI(blunted) fragment (710bp) was purified by agarose gel electrophoresis and elution from a gel slice and ligated with SmaI-digested, phosphatased pXY460. The DNA was transformed into E. coli strain TG1 and recombinants selected as blue colonies on L-Amp-Xgal plates. Individual transformants were characterised by restriction enzyme digestion and one of those which had the predicted pattern of fragments was called pDM614. Transformants were also characterised by their immunoreactivity with sera from AIDS patients. The gag sequence incorporated in pDM614 is shown in Figure 2.

c) Gag/env

The HIV-1 sequences expressed individually in pDM322 and pDM614 were combined together in the following manner. In pDM614 a SmaI site was retained at the 3′ end of the gag insert because the filled-in NciI site represents "half" a SmaI site (CCCGGG). When the EcoRI site of pDM322 was filled-in using the Klenow fragment of E. coli DNA polymerase I, the resulting blunt end was in-frame with the SmaI site of pDM614.

```
                    DM324 DM322
    gag ... GCA GGA CCC AAT TCC CCC AGA CAA ... env
            Gly Gly Pro Asn Ser Pro Arg Gln
```

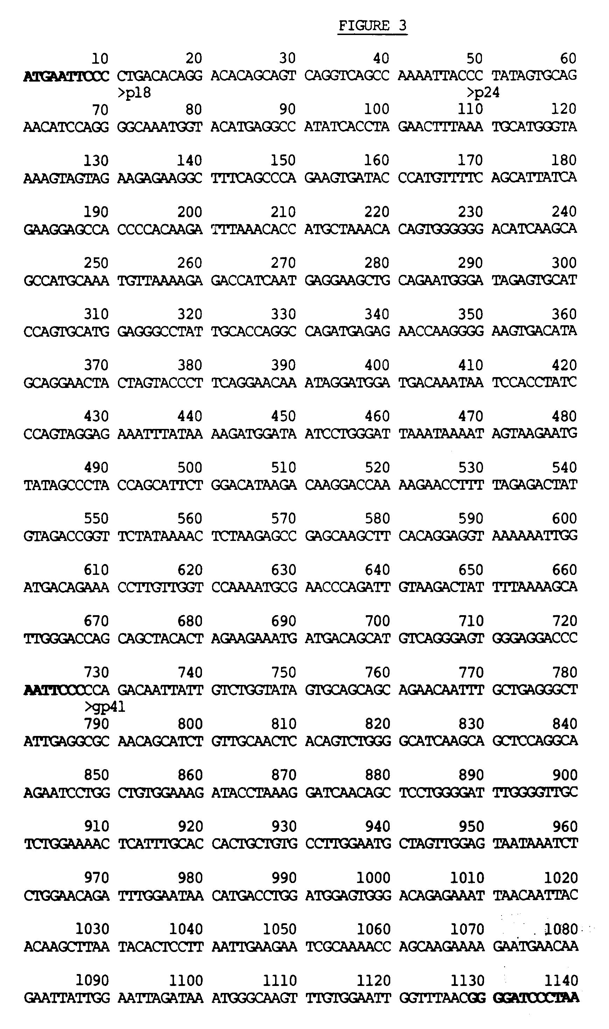

The plasmid DM322 was digested with EcoRI, filled-in and then digested with BamHI; the resulting 410bp fragment was gel-purified. The plasmid pDM614 was digested with SmaI and BamHI and gel purified. The two fragments were ligated together and transformed into E. coli TG1. The blue colonies selected on L-Amp-Xgal plates were analysed by restriction enzyme digestion. One transformant which had the predicted pattern of digestion fragments was called pDM624. The 1120bp EcoRI/BamHI fragment from pDM624 was also transferred to the plasmid pXY46X, which was derived from pXY460 by deletion of all of the lacZ gene and the insertion of in-frame termination codons next to the BamHI site, to produce pDM626. The DNA sequence of the gag/env fusion of pDM626 is shown in Figure 3.

d) Antigen production

The recombinant E. coli strains were plated onto selective media (L-Amp or L-Amp-Xgal) and single colonies used to inoculate overnight cultures (<300ml). Portions of these inocula were added to larger volumes of medium (up to 3 litres) in fermentation vessels. The growth of the cultures was monitored and when the $OD_{600}$ reached 2.0-2.5 the inducer IPTG (isopropyl-$\beta$-D-thiogalactopyranoside) was added to a final concentration of 5$\mu$g/ml. The bacteria were then grown for a further 2-4 hours to allow induction of the recombinant proteins.

The cells were harvested by centrifugation and resuspended in buffer (25mM Tris-Cl, pH8.0, 1 mM EDTA, 0.2% Nonidet P40) to a final concentration of 100 $OD_{600}$ units. Extracts were prepared from the cells by one of two methods.

i. Lysozyme and PMSF were added to the resuspended cells to final concentrations of 1 mg/ml and 1 mmol respectively and incubated overnight at 4°C. $MgSO_4$ (2mM) and DNaseI (40 $\mu$g/ml) were added

and incubation continued at 4°C. More PMSF was added to 2 mM and EDTA was adjusted to 5 mM. The extract was clarified by centrifugation at 15000g for 20 minutes. The supernatant was decanted and retained at -70°C.

ii. The resuspended cells were passed through a French-pressure cell at an operating pressure of 12-15000 psi. PMSF and EDTA were added to the lysate to final concentrations of 2 mM and 5mM respectively. The extract was clarified by centrifugation at 15000g for 20 minutes. The supernatant was decanted and stored at -70°C. The passages through the pressure cell may be repeated to increase the release of antigen from the cells.

The env/β-gal and gag/β-gal fusion proteins were purified by affinity chromatography making use of the β-galactosidase enzyme protein engineered into them, either on an anti-galactosidase affinity column or on a substrate-affinity column. Following this procedure the antigens may be further purified on a size exclusion column, and are then homogeneous by analytical gel-electrophoresis.

The gag/env fusion protein was purified from the insoluble fraction of lysed cells by differential extraction with a chaotropic agent (urea). The fraction soluble between 3 and 8M urea was further purified by chromatography on a phenyl Sepharose® column with elution in 8M urea. The overall yield was greater than 70% of antigenic activity and greater than 80% of the protein in bands immunologically reacting as fusion proteins subsequent to electrophoresis (SDS-PAGE) and Western blotting.

Example 2. Baculovirus expression constructions

The baculovirus Autographa californica nuclear polyhedrosis virus (AcNPV) has been developed into a useful helper-independent eukaryotic expression vector (Rohrnann (1986) J. Gen. Virol. 67 1499-1513, Kuroda et al (1986) EMBO J. 5 No.6 1359-1365). The vector makes use of the polyhedrin gene of the virus which has two attractive properties. It is highly expressed late in the virus life-cycle. It is not essential for virus growth. Foreign genes are introduced into the polyhedrin gene so that they are expressed under the control of its promoter and other regulatory elements and the polyhedrin gene is itself inactivated. The AcNPV genome is very large and cannot be used for direct cloning; instead a transfer vector must be used. The transfer vector contains the polyhedrin gene and some flanking sequences, in an E. coli plasmid such as pUC8, with convenient restriction sites introduced into the gene. When the transfer vector is introduced into insect cells (e.g. Spodoptera frugiperda, FP9) which are also infected with AcNPV recombination can occur. The recombinants, which occur at a frequency of less than 1%, have replaced the wild-type polyhedrin gene with one which now expresses the foreign gene; such viruses are polyhedrin -minus and can be identified by their plaque morphology. We have expressed a truncated form of HIV-1 envelope protein and the gag/env protein in this system.

a) Envelope

The DraI site (AATATT) at position 6190 is 65bp upstream of the start of the HIV-1 env gene. This site was converted to BamHI by cloning into the vector pUC9 to produce pDM100.

```
... TAGGAAAATATTAAGACA ....→ ....GGATCCCCATTAAGAGA.......
           DraI                     BamHI
```

There is a BamHI site within the env gene at position 8505 which truncates the coding sequence at amino acid 752.

```
                    750  BamHI



                ... GTG AAC GGA TCC TTA
                    Val Asn Gly Ser Leu
```

The 2.32Kb BamHI fragment from pDM100, purified by agarose gel electrophoresis as above, was ligated with the so-called baculovirus transfer vector pAc373 (Kuroda et al) and transformed into E. coli strain HB101. A transformant which had the env insert in the correct orientation for expression from the polyhedrin promoter was identified by restriction enzyme mapping. This was then transfected into SP9 cells infected with AcNPV. Polyhedrin -minus viruses were identified and plaque purified by standard techniques (Summers and Smith "Manual of Methods for Baculovirus Vectors and Insect Culture Procedures" (1986) Texas Agricultural Experimental Station; "Current Topics in Microbiology and Immunology", Number 131, The Molecular Biology of Baculoviruses, Year: 1986, Editos: W. Doerfler and P. Bohm, Publishers: Springer Verlag).

b) Gag/env

The 1120bp EcoRI/BamHI fragment of pDM626 cannot be expressed directly using the pAc373 vector because it does not have its own initiating ATG codon. The EcoRI site at the 5' end of the fragment was modified by the addition of a synthetic oligonucleotide which provided an ATG and converted the EcoRI site to that for BglII.

```
5'   AATTCAT AGATCT ATG 3'
       3' GTA TCTAGA TACTTAA 5'
                  BglII
```

The gag/env coding sequence was cloned into BamHI-digested pAc373 as a BglII/BamHI fragment and recombinant AcNPV generated as described above.

c) Preparation of antigens

The recombinant AcNPV were used to infect fresh cultures of SP9 cells at a multiplicity of infection of 1-10. The infected cells were maintained in stirred flasks at 28°C for 36-72 hours, at which time the cells were harvested and lysed at $2\text{-}5 \times 10^7$ cells/ml with 1% Nonidet NP40 to release the antigen. The antigen was clarified by centrifugation at $15000g_{av}$ for 20 minutes at 4°C. The supernatant was stored at -70°C until required. The antigen may be purified by affinity chromatography on lentil lectin columns. Antigens prepared in this fashion are significantly purified over the cell lysate but are not analytically pure.

Example 3: Vaccinia virus expression constructions

Two clones were used to construct fusion proteins composed of the major antigenic epitope of foot-and-mouth disease virus (FMDV) fused to the amino-terminus of hepatitis B core antigen (HBcAg). One clone representing HBcAg was obtained from Dr. P. Highfield (pWRL 3123). This clone had been modified at the $NH_2$ terminus such that it could be expressed in bacteria as a fusion protein to the E. coli protein TRP E. pWRL 3123, harbored in E. coli HB101, was deposited at NCIB, Aberdeen, GB on 6 March 1987 under accession number NCIB 12423.

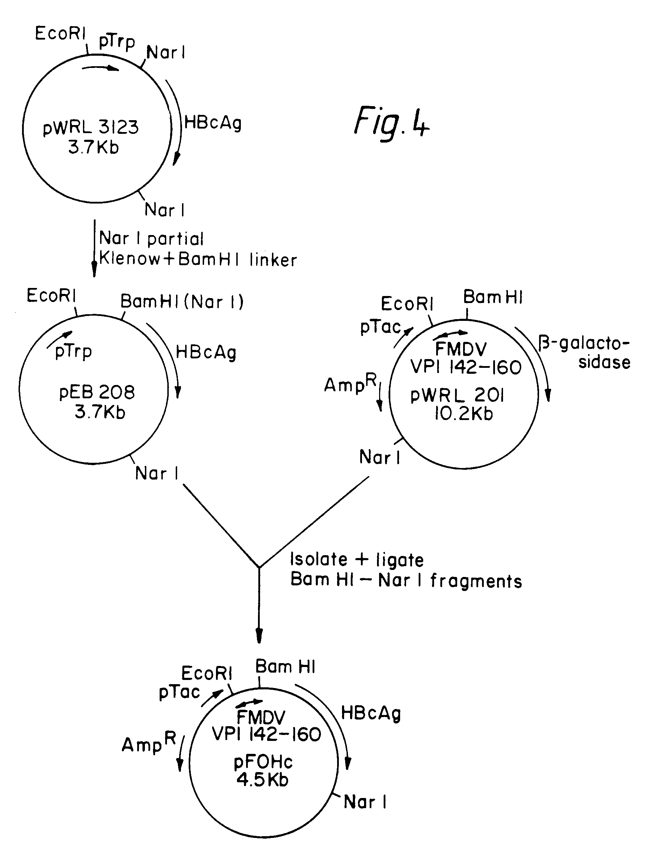

A second clone representing FMDV VP1 142-160 sequences from $0_1$ Kaufbeuren linked to the amino terminus of β-galactosidase was obtained from Dr. M. Winther (pWRL 201) (Winther et al, J. Immunol. 136, 1835, 1986). Restriction maps of each clone are shown in Figure 4. As can be seen in Figure 4, the junction between the FMDV sequence and the β-galactosidase comprises a BamHI restriction site. The strategy undertaken therefore involved the fusion of the FMDV sequence and the HBcAg sequence through this BamHI site.

The initial stage in the construction therefore involved insertion of a synthetic oligonucleotide linker for BamHI at the 5' end of the HBcAg gene of pWRL 3123. The site used for insertion of the linker was the NarI site at position 290. However a second NarI site at position 1230 was also present in this plasmid. The plasmid was therefore partially digested with NarI so that a population of plasmid molecules which had been cut at only one NarI site could be observed by agarose gel electrophoresis and purified. After flush ending the NarI sites using the Klenow fragment of DNA polymerase I, a synthetic oligonucleotide linker representing a BamHI site was ligated into the partial NarI digest and the resulting plasmids were used to transform E. coli. Clones were then analysed for the presence of a BamHI linker in the correct NarI site by restriction mapping.

One such clone, designated pEB208, was isolated and DNA prepared. The length of the BamHI linker had been specifically chosen so that, when ligated to the FMDV portion of pWRL201 (Fig 4), the translational reading frame would be continuous and a fusion protein could be produced. Concomitant with the insertion of the BamHI linker, the NarI site into which it had been inserted was destroyed. It was therefore possible to remove the HBcAg sequence from pEB208 by BamHI - NarI digestion whereupon a DNA fragment of 940 bases was produced. Similarly a BamHI - NarI fragment from pWRL201 of approximately 3.5 kilobases purified. These two fragments were ligated together and the correct clone (pFOHc) was identified by restriction mapping.

As can be seen from Figure 4, pFOHc can be expressed in bacterial cells under the control of the tac promoter. In order to facilitate the transfer of the hybrid gene to a vaccinia virus (VV) shuttle vector, however, plasmid pFOHc was cut at the single NarI site and a second EcoRI site was introduced as a synthetic linker. This enabled the complete hybrid gene to be isolated as an EcoRI fragment.

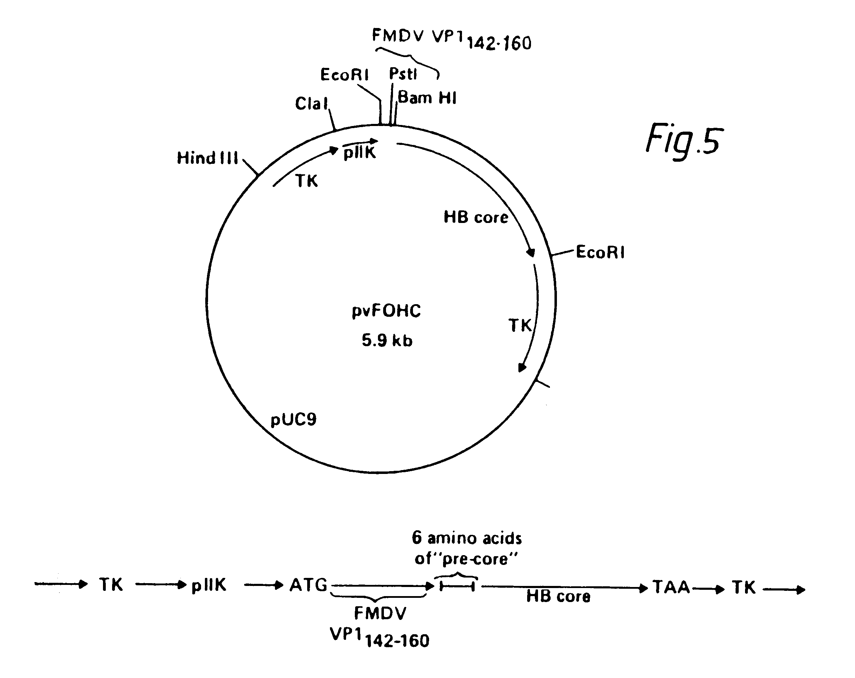

The VV shuttle vector was pVp11k which was derived from from the vector pH3JΔR1A (Newton et al, Vaccines 86: New Approaches to Immunisation, Cold Spring Harbor Laboratory, 303-309, 1986) by deletion of extraneous VV sequences. This shuttle vector has a VV promoter (in this case p11K) inserted into VV thymidine kinase (TK) gene. This vector has a unique EcoRI site immediately following the VV p11k promoter and AUG (Bertholet et al, PNAS, 82, 2096, 1985). The EcoRI site and AUG are in the same translational reading frame as the amino terminal EcoRI site of the hybrid gene in pFOHc. The FMDV-HBcAg gene was therefore inserted as the EcoRI fragment into EcoRI cut dephosphorylated pVp11k. Clones with the hybrid gene in the correct orientation relative to the p11k promoter were identified by restriction mapping. This clone was designated pvFOHc (Figure 5).

This shuttle plasmid was then inserted into the genome of the Wyeth (US vaccine) strain of VV, under the control of the p11k promoter, by homologous recombination using the flanking TK sequences. Individual progency plaques with a TK⁻ phenotype were screened for the presence of FMDV-HBcAg DNA by dot blot hybridisation.

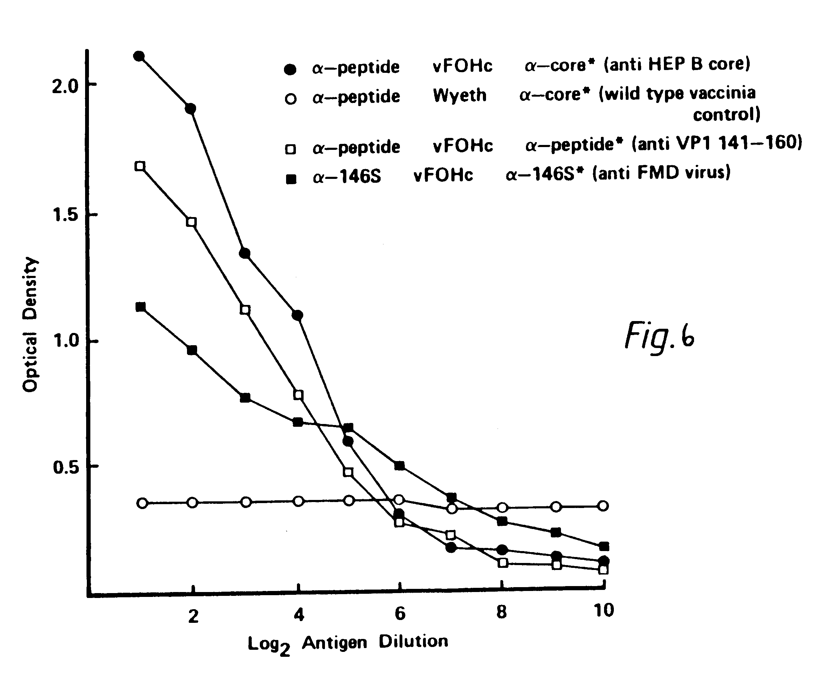

CV-1 Cell lysates from wild-type (Wyeth) and recombinant (vFOHc) infected cells were screened for the presence of core antigen and for FMDV sequences by sandwich ELISA. Antigen from infected cells was bound to ELISA plates using either FMD virus particle (146S) or FMD VP1 141-160 antisera raised in rabbits. Each trapped antigen was then assessed for the presence of either HBc, FMD 146S or FMD VP1 142-160 epitopes by binding with the respective guinea pig antisera and development with anti guinea pig peroxidase conjugate. The results are shown in Figure 6. As can be seen in Fig 6, a protein recombinant from (vFOHc) infected cell lysates was trapped with anti-FMDV 141-160 antiserum and this protein could then react with anti HBc, anti-FMDV 141-160 and FMDV antivirion serum in a sandwich ELISA.

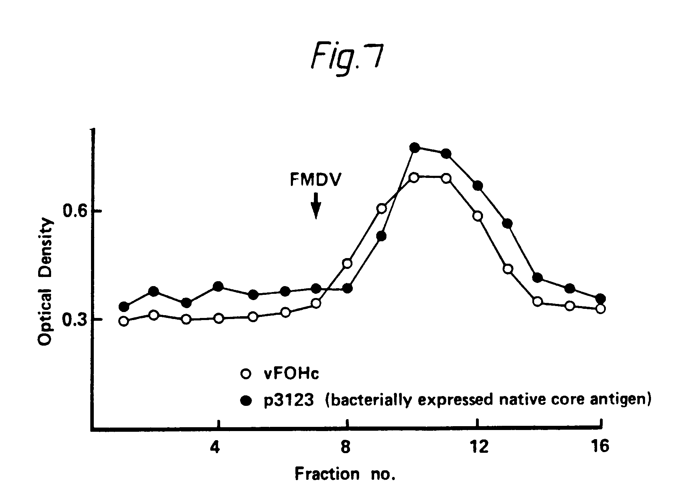

Furthermore, this protein could be purified by ultracentrifugation suggesting that it was particulate in nature. This was illustrated more clearly when the products of centrifugation were sedimented on a sucrose density gradient and fractions were re-assayed for the presence of core antigen by ELISA Cell lysates from recombinant (vFOHc) vaccinia virus infected cells or bacteria expressing native core antigen were fractionated on 15-45% sucrose gradients. Fractions were assayed for the presence of core reactive material by indirect sandwich ELISA using human anticore antiserum as trapping antibody and guinea pig HBc antigen antiserum for detection. The results are shown in Figure 7. The position at which FMD virus sediments is also indicated. Fig 7 shows that a peak of HBcAg reactive material was observed in a position similar to that observed when core particles expressed in bacteria were centrifuged in parallel. Thus it appears that the presence of the FMDV VP1$_{142-160}$ sequence does not interfere with the particulate nature of the core particles.

The ability of the fusion protein to self assemble into regular, 27nm core like particles was confirmed by electron microscopic examination of immune complexes formed with sucrose gradient purified material. The complexes were formed by reacting the FMDV-HBcAg particles with antiserum raised to intact foot and mouth disease virus. The complexes were adsorbed to form over coated grids and negatively stained with phosphotungstic acid. As was to be expected from the ELISA data shown in Fig 6, immune complexes were also seen after reacting the particles with antisera to HBcAg or to synthetic FMDV peptide 141-160.

Example 4: Assay using recombinant HIV antigens

a) Preparation of coated microwells

The antigens obtained from insect cells in Example 2 were coated at a predetermined optimum concentration onto the microwells by passive adsorption from an amine-containing buffer at pH 8. The wells were then supercoated with a solution containing high levels of a bovine protein to ensure that any remaining hydrophobic sites are fully occupied.

15

b) Preparation of conjugates

1. The purified antigens from E. coli from Example 1 were labelled with alkaline phosphatase. The alkaline phosphatase was attached to the sulphydryl radicals of the $\beta$-galactosidase using well established maleimide-sulphydryl chemistry.

2. The conjugates were freeze-dried in a matrix of a sugar alcohol with serum protein additives, and reconstituted before use with a diluent containing the metal cofactors of alkaline phosphatase.

c) Performing an assay

1. Sequential format.

A sample to be assayed for the presence of anti-HIV antibodies was added to a microwell and incubated for 30 minutes at a temperature of 45°C. The residues were washed from the microwell and then the conjugate was added to the well and incubated for 30 minutes at a temperature of 45°C. The excess conjugate was removed by washing. Then the presence of alkaline phosphatase was detected using the cyclic amplification system described previously. Any significant amount of enzyme bound to the well indicated the presence in the sample of antibodies to the envelope proteins of HIV.

The assay was tested using 1662 sera known not to contain antibodies to the envelope proteins of HIV, and with 6 sera containing such antibodies. The results are shown in Table 1 ("Insect cell cultured antigen"), with for comparison an assay performed with the antigens derived from E.coli both as conjugate and as coating protein ("E. coli cultured antigen"). It can be seen that the background colouration is higher in the latter case and that there occur samples which give rise to signals which can be ascribed to impurities in the antigen preparations and not to the presence of genuine anti-HIV antibodies. These samples do not give rise to signals on those assays wherein the coating and conjugated antigens are prepared from different sources.

Table 1

(a) Backgrounds generally
Insect cell cultured antigen : assay background = 0.15 ± 0.03 O.D. Units
E. coli cultured antigen : assay background = 0.26 + 0.05
(b) False positive data (ascribable to anti-coli activity)
E. coli cultured antigen : 2 false positive signals from
1662 samples
Not reactive on Insect cell cultured antigen
(c) Positives found as positive using Insect cell cultured antigen 6/6

Example 5: Detection of antibody against HIV by competition for binding of a labelled antibody itself directed to the gag/env protein of the invention

A classic competitive enzyme immunoassay (EIA) was used. The gag/env fusion protein, designated here 626, obtained in Example 1 was coated onto microtitre wells by capture through a monoclonal antibody (TLO3) directed against p24. Samples were then added to the prepared wells, and a conjugated anti-HIV added immediately. The samples were serum samples and plasma samples from blood donors and serum samples from patients with AIDS, AIDS-associated condition and other diseases. The enzyme used in the conjugate was peroxidase. After an incubation period of about 1 hour the wells were washed and substrate for the enzyme was added. This was 3,3,5,5′-tetramethyl benzidine. Anti-HIV in the sample was ascertained by comparison with a standard taken through the procedure. The results are given in Tables 2 and 3.

EP 0 307 149 B1

Table 2

| Detection of antibody to HIV in serum samples and plasma samples from blood donors | | | | |
|---|---|---|---|---|
| Centre | No. samples tested | Non-reactive | Initially reactive | Repeatably reactive |
| 1 | 1699 | 1696 | 3 | 1 (0.06%) |
| 2 | 1783 | 1780 | 3 | 2 (0.11%) |
| 3 | 2037 | 2034 | 3 | 1 (0.05%) |
| 4 | 1908 | 1904 | 4 | 2* (0.10-0.16%) |
| 5 | 975 | 974 | 1 | 0 |
| Total | 8402 | 8388 | 14 (0.17%) | 6 (0.07%) |

* Only 3/4 samples retested

## Table 3: Reactivity of sera from patients with AIDS, AIDS associated condition and other diseases.

| Clinical Group | No. of Samples | Antibody Positive | Confirmed Antibody Positive[a] |
|---|---|---|---|
| AIDS | 59 | 59 | 59[b] |
| AIDS related complex | 62 | 62 | 62 |
| AIDS associated conditions[c] | 97 | 97 | 97 |
| High risk[d] | 426 | 272[+] | 271 |
| Diseases unrelated to AIDS[e] | 67 | 1[++] | 0 |

17

Miscellaneous[f]          80              0              0

[a]    Confirmation was by Western Blot and/or at least two alternative immunoassays (except [b] below).

[b]    Samples from 30 AIDS patients were confirmed with one alternative immunoassay.

[c]    Includes patients with Persistent Generalised Lymphadenopathy, Kaposi's sarcoma, opportunistic infections and patients known to be HIV antibody positive.

[d]    Patients in established risk groups.

[e]    Patients with acute viral diseases, autoimmune disease, neoplasia.

[f]    Includes samples from healthy individuals and patients with undefined conditions.

[+]    The discrepant sample (from an IV drug abuser) gave an uninterpretable Western blot.

[++]   The discrepant sample was grossly haemolysed.

Example 6: Detection of antibody against HIV by labelling with conjugated anti-globulin

This is the standard method of detecting antibodies to HIV. The gag/env fusion polypeptide from plasmid pDM 626 page 29 below from Example 1 was coated onto microwells by passive adsorption. Samples of 50ul of serum, pre-diluted 1/100, were added to the wells. After an incubation of about 30 minutes the samples were washed out of the wells and 50ul conjugated anti-human globulin added. The enzyme used in the conjugate was peroxidase. After incubation for a further 30 minutes approximately, the wells were again washed and enzyme substrate added. The presence of anti-HIV was detected by comparison with a standard taken through the procedure. The results are given in Table 4.

Table 4

| Indirect anti-globulin test | | | |
|---|---|---|---|
| Number Negatives Tested | Number Negative | Number Positives Tested | Number Positive |
| 55 | 55 | 25 | 25 |

Example 7: Detection of antibody against HIV by use of labelled 626

626 from Example 1 was coated passively onto microwells as in Example 6. Undiluted 250ul samples were added to the prepared wells. Conjugated 626 (50ul) was added immediately after samples. After an incubation for about an hour the wells were washed and substrate added. The enzyme used was alkaline phosphatase. The results are shown in Table 5.

Table 5

| Direct sandwich assay | | | | |
|---|---|---|---|---|
| | Number Negatives Tested | Number Negative | Number Positives Tested | Number Positive |
| Sera | 810* | 809 | 58 | 169 |
| Plasma | 175* | 169 | | |

* 1 false positive sera and 6 false positive plasmas (not all repeatably false positive)

Example 8: Detection of captured antibody against HIV using 626 labelled with a particle

A standard agglutination test was effected. Latex particles of a diameter of 0.2 micron precoating were coated passively with 626 obtained in Example 1. Samples of sera or plasma were mixed with the latex either using an apparatus or by stirring on a surface. The presence of antibodies to HIV (which cause agglutination of the particles) was ascertained by visual inspection, or by appropriate instrumentation, a few minutes after mixing the reagents. The results are shown in Table 6.

Table 6

| Latex Agglutination: Assay | | | | |
|---|---|---|---|---|
| | Number Negatives Tested | Number Negative | Number Positives Tested | Number Positive |
| Sera | 480 | 479 | 80 | 80 |
| Plasma | 50 | 50 | | |

Example 9: Use of 626 to label anti-HIV antibody captured by anti-globulin

Anti-human globulin was coated passively onto microwells. Samples each of 50ul of undiluted serum were added to the prepared wells. 626 from Example 1 labelled with alkaline phosphatase was added immediately. After an incubation of about one hour the wells were washed and substrate added. The presence of anti-HIV antibody was ascertained by comparison with a standard. The results are shown in Tables 7 and 8.

Table 7

| Anti-human capture assay | | | |
|---|---|---|---|
| Number Negatives Tested | Number Negative | Number Positives Tested | Number Positive |
| 16 | 16 | 15* | 12 |

* includes 3 weak positives not detected

Table 8

| Anti-human capture assay | | | |
|---|---|---|---|
| Number Negatives Tested | Number Negative | Number Positives Tested | Number Positive |
| 138* | 137 | 46** | 45 |

* 1 false positive
** very weak positive not detected

**Claims**

1. An immunoassay for an antibody, which immunoassay comprises:
   (i) contacting a solid phase, on which is immobilised a first recombinant peptide which presents an antigenic sequence to which the antibody is capable of binding, with a test sample;
   (ii) contacting the solid phase with a second recombinant peptide which presents the said antigenic sequence and which is labelled; and
   (iii) determining whether the test sample contained any said antibody;
   characterised in that the second recombinant antigen was expressed in an organism of a different genus than that in which the first recombinant peptide was expressed.

2. An immunoassay according to claim 1, wherein one of the recombinant peptides was expressed in a procaryotic organism whilst the other was expressed in a eucaryotic organism.

3. An immunoassay according to claim 2, wherein one of the recombinant peptides was expressed in E. coli and the other was expressed in insect cells or mammalian cells by way of a baculovirus or vaccinia virus expression system respectively.

4. An immunoassay according to any one of the preceding claims, wherein the recombinant peptides are HIV gag and/or end sequences.

5. A test kit for use in an immunoassay for an antibody, which kit comprises:
   (a) a solid phase on which is immobilised a first recombinant peptide which presents an antigenic sequence to which the antibody is capable of binding; and
   (b) a second recombinant peptide which presents the said antigenic sequence and which is labelled;
   characterised in that the second recombinant antigen was expressed in an organism of a different genus than that the first recombinant peptide was expressed.

6. A protein of the sequence:

```
                         10                                    20
MetAsnSerProAspThrGlyHisSerSerGlnValSerGlnAsnTyrProIleValGln
         p18>                                       p24>

                         30                                    40
AsnIleGlnGlyGlnMetValHisGlnAlaIleSerProArgThrLeuAsnAlaTrpVal

                         50                                    60
LysValValGluGluLysAlaPheSerProGluValIleProMetPheSerAlaLeuSer

                         70                                    80
GluGlyAlaThrProGlnAspLeuAsnThrMetLeuAsnThrValGlyGlyHisGlnAla

                         90                                   100
AlaMetGlnMetLeuLysGluThrIleAsnGluGluAlaAlaGluTrpAspArgValHis

                        110                                   120
ProValHisAlaGlyProIleAlaProGlyGlnMetArgGluProArgGlySerAspIle

                        130                                   140
AlaGlyThrThrSerThrLeuGlnGluGlnIleGlyTrpMetThrAsnAsnProProIle

                        150                                   160
ProValGlyGluIleTyrLysArgTrpIleIleLeuGlyLeuAsnLysIleValArgMet

                        170                                :  180
TyrSerProThrSerIleLeuAspIleArgGlnGlyProLysGluProPheArgAspTyr
```

```
                              190                                200
          ValAspArgPheTyrLysThrLeuArgAlaGluGlnAlaSerGlnGluValLysAsnTrp

                              210                                220
          MetThrGluThrLeuLeuValGlnAsnAlaAsnProAspCysLysThrIleLeuLysAla

                              230                                240
          LeuGlyProAlaAlaThrLeuGluGluMetMetThrAlaCysGlnGlyValGlyGlyPro

                              250                                260
          AsnSerProArgGlnLeuLeuSerGlyIleValGlnGlnGlnAsnAsnLeuLeuArgAla
                gp41>

                              270                                280
          IleGluAlaGlnGlnHisLeuLeuGlnLeuThrValTrpGlyIleLysGlnLeuGlnAla

                              290               .                300
          ArgIleLeuAlaValGluArgTyrLeuLysAspGlnGlnLeuLeuGlyIleTrpGlyCys

                              310                                320
          SerGlyLysLeuIleCysThrThrAlaValProTrpAsnAlaSerTrpSerAsnLysSer

                              330                                340
          LeuGluGlnIleTrpAsnAsnMetThrTrpMetGluTrpAspArgGluIleAsnAsnTyr

                              350                                360
          ThrSerLeuIleHisSerLeuIleGluGluSerGlnAsnGlnGlnGluLysAsnGluGln

                              370               .
          GluLeuLeuGluLeuAspLysTrpAlaSerLeuTrpAsnTrpPheAsnGlyAspPro;
```

optionally modified by one or more amino acid substitutions, insertions and/or deletions and/or by an extension at either or both ends provided that a protein having such a modified sequence is capable of binding to both anti-p24 and anti-gp41 and there is a degree of homology of at least 75% between the modified and the unmodified sequences.

**7.** A process for the preparation of a recombinant protein by
   (i) transforming a host cell with a vector which incorporates a gene encoding the protein and which is capable, in the host cell, of expressing the protein;
   (ii) culturing the transformed host cell so that the protein is expressed; and
   (iii) recovering the protein;
   characterised in that the said gene encodes a protein as claimed in claim 6.

**8.** An assay for anti-p24 and/or anti-gp41 HIV-1 antibody, which assay comprises contacting a test sample with a protein capable of binding to anti-p24 and/or anti-gp41 HIV-1 antibody and determining whether any of the said antibody binds to the protein, characterised in that the protein is a protein as claimed in claim 6.

**9.** A test kit for use in an assay for anti-p24 and/or anti-gp41 HIV-1 antibody, which kit comprises a protein capable of binding to anti-p24 and/or anti-gp41 HIV-1 antibody and means for determining whether any of the said antibody in a test sample binds to the protein, characterised in that the protein is a protein as claimed in claim 6.

**10.** A pharmaceutical composition comprising a pharmaceutically acceptable carrier or diluent and, as active ingredient, a protein as claimed in claim 6.

**Patentansprüche**

1. Immuntest für einen Antikörper, umfassend die Schritte:

(i) Inkontaktbringen einer festen Phase, auf welches ein erstes rekombinantes Peptid, die eine antigenische Sequenz, gegen die der Antikörper zur Bindung in der Lage ist, präsentiert, immobilisiert ist, mit einer Probe;

(ii) Inkontaktbringen der festen Phase mit einem zweiten rekombinanten Peptid, welches die antigenische Sequenz präsentiert und die markiert ist; und

(iii) Nachweis, ob die Testprobe den Antikörper enthält,

dadurch charakterisiert, daß das zweite rekombinante Antigen in einem Organismus von einer anderen Gattung als der experimentiert wurde, in welcher das erste rekombinante Peptid exprimiert wurde.

2. Immuntest nach Anspruch 1, worin eines der rekombinanten Peptide in einem prokaryntischen Organismus exprimiert wurde, während das andere in einem eukaryontischen System exprimiert wurde.

3. Immuntest nach Anspruch 1, worin eines der rekombinanten Peptide in E. coli exprimiert wurde, und das andere in Insektenzellen oder Säugerzellen mittels eines Baculovirus- oder Vacciniavirus-Expressionssystems exprimiert wurde.

4. Immuntest nach einem der vorhergehenden Ansprüche, worin die rekombinanten Peptide HIV-, gag- und/oder env-Sequenzen sind.

5. Testkit zur Verwendung in einem Immuntest für einen Antikörper, umfassend:

(a) eine feste Phase, auf welche ein erstes rekombinantes Peptid immobilisiert ist, welches eine antigenische Sequenz, gegen die der Antikörper zur Bindung in der Lage ist, präsentiert; und

(b) ein zweites rekombinantes Peptid, welches die antigenische Sequenz präsentiert und markiert ist,

dadurch gekennzeichnet, daß das zweite rekombinante Antigen in einem Organismus von einer anderen Gattung exprimiert wurde als der, in welcher das erste rekombinante Peptid exprimiert wurde.

6. Protein der Sequenz:

```
                              10                                           20
  MetAsnSerProAspThrGlyHisSerSerGlnValSerGlnAsnTyrProIleValGln
            p18>                                         p24>

                              30                                           40
    AsnIleGlnGlyGlnMetValHisGlnAlaIleSerProArgThrLeuAsnAlaTrpVal

                              50                                           60
  LysValValGluGluLysAlaPheSerProGluValIleProMetPheSerAlaLeuSer

                              70                                           80
    GluGlyAlaThrProGlnAspLeuAsnThrMetLeuAsnThrValGlyGlyHisGlnAla

                              90                                          100
    AlaMetGlnMetLeuLysGluThrIleAsnGluGluAlaAlaGluTrpAspArgValHis

                             110                                          120
    ProValHisAlaGlyProIleAlaProGlyGlnMetArgGluProArgGlySerAspIle

                             130                                          140
    AlaGlyThrThrSerThrLeuGlnGluGlnIleGlyTrpMetThrAsnAsnProProIle

                             150                                          160
  ProValGlyGluIleTyrLysArgTrpIleIleLeuGlyLeuAsnLysIleValArgMet

                             170                                          180
    TyrSerProThrSerIleLeuAspIleArgGlnGlyProLysGluProPheArgAspTyr
```

24

```
                    190                                    200
ValAspArgPheTyrLysThrLeuArgAlaGluGlnAlaSerGlnGluValLysAsnTrp

                    210                                    220
MetThrGluThrLeuLeuValGlnAsnAlaAsnProAspCysLysThrIleLeuLysAla

                    230                                    240
LeuGlyProAlaAlaThrLeuGluGluMetMetThrAlaCysGlnGlyValGlyGlyPro

                    250                                    260
AsnSerProArgGlnLeuLeuSerGlyIleValGlnGlnGlnAsnAsnLeuLeuArgAla
          gp41>

                    270                                    280
IleGluAlaGlnGlnHisLeuLeuGlnLeuThrValTrpGlyIleLysGlnLeuGlnAla

                    290            .                       300
ArgIleLeuAlaValGluArgTyrLeuLysAspGlnGlnLeuLeuGlyIleTrpGlyCys

                    310                                    320
SerGlyLysLeuIleCysThrThrAlaValProTrpAsnAlaSerTrpSerAsnLysSer

                    330            ,                       340
LeuGluGlnIleTrpAsnAsnMetThrTrpMetGluTrpAspArgGluIleAsnAsnTyr

                    350                                    360
ThrSerLeuIleHisSerLeuIleGluGluSerGlnAsnGlnGlnGluLysAsnGluGln

                    370            .
GluLeuLeuGluLeuAspLysTrpAlaSerLeuTrpAsnTrpPheAsnGlyAspPro ;
```

die wahlweise durch eine oder mehr Aminosäuresubstitutionen, Insertionen und/oder Deletionen und/oder durch Verlängerung an einer oder beiden Enden modifiziert ist, vorausgesetzt, daß ein Protein mit einer solchen modifizierten Sequenz zur Bindung an sowohl anti-p24 und anti-gp41 in der Lage ist und daß ein Ausmaß an Homologie von mindestens 75 % zwischen den modifizierten und den unmodifizierten Sequenzen besteht.

7. Verfahren zur Herstellung eines rekombinanten Proteins durch
(i) Transformation einer Wirtszelle mit einem Vektor, welcher ein Gen, das für das Protein kodiert, enthält und das zur Expression des Gens in der Wirtszelle in der Lage ist;
(ii) Kultur der transformierten Wirtszelle, so daß das Protein exprimiert wird und
(iii) Gewinnen des Proteins,
dadurch gekennzeichnet, daß das Gen ein Protein gemäß Anspruch 6 kodiert.

8. Test auf anti-p24 und/oder anti-gp41 HIV-1-Antikörper, umfassend die Schritte: Inkontaktbringen einer Probe mit einem Protein, das zur Bindung an anti-p24- und/oder anti-gp41-HIV-1-Antikörper in der Lage ist und Bestimmen, ob einer der Antikörper an das Protein bindet, dadurch gekennzeichnet, daß das Protein ein Protein gemäß Anspruch 6 ist.

9. Testkit zur Verwendung in einem Test auf anti-p24- und/oder anti-gp41-HIV-1-Antikörper, wobei das Kit ein Protein, das zur Bindung an anti-p24- und/oder anti-gp41-HIV-1-Antikörper in der Lage ist und Mittel zum Nachweis enthält, ob eines der Antikörper in der Probe an das Protein bindet, dadurch gekennzeichnet, daß das Protein ein Protein gemäß Anspruch 6 ist.

10. Pharmazeutische Zusammensetzung, enthaltend einen pharmazeutisch annehmbaren Träger oder Diluens und als wirksamen Bestandteil ein Protein gemäß Anspruch 6.

**Revendications**

1.  Immunodosage d'un anticorps, lequel immunodosage comprend :
    (i) la mise en contact d'une phase solide, sur laquelle est immobilisé un premier peptide recombinant qui présente une séquence antigénique sur laquelle l'anticorps est capable de se lier, avec un échantillon à examiner;
    (ii) la mise en contact de la phase solide avec un deuxième peptide recombinant qui présente cette séquence antigénique et qui est marqué, et
    (iii) la détermination du fait que l'échantillon examiné contient ou non l'anticorps;
    caractérisé en ce que le deuxième antigène recombinant a été exprimé dans un organisme appartenant à un genre différent de celui dans lequel le premier peptide recombinant a été exprimé.

2.  Immunodosage suivant la revendication 1, dans lequel l'un des peptides recombinants a été exprimé dans un organisme procaryote, tandis que l'autre a été exprimé dans un organisme eucaryote.

3.  Immunodosage suivant la revendication 2, dans lequel l'un des peptides recombinants a été exprimé dans E. coli et l'autre a été exprimé dans des cellules d'un insecte ou des cellules d'un mammifère à l'aide d'un système d'expression avec un baculovirus ou avec le virus de la vaccine, respectivement.

4.  Immunodosage suivant l'une quelconque des revendications précédentes, dans lequel les peptides recombinants sont les séquences gag et/ou env de HIV.

5.  Trousse d'essai à utiliser dans un immunodosage d'un anticorps, laquelle trousse comprend :
    (a) une phase solide sur laquelle est immobilisé un premier peptide recombinant qui présente une séquence antigénique sur laquelle l'anticorps est capable de se lier, et
    (b) un deuxième peptide recombinant qui présente la séquence antigénique et qui est marqué;
    caractérisée en ce que le deuxième antigène recombinant a été exprimé dans un organisme appartenant à un genre différent de celui dans lequel le premier peptide recombinant a été exprimé.

6.  Protéine de la séquence :

```
                      10                                    20
MetAsnSerProAspThrGlyHisSerSerGlnValSerGlnAsnTyrProIleValGln
            p18>                                  p24>

                      30                                    40
AsnIleGlnGlyGlnMetValHisGlnAlaIleSerProArgThrLeuAsnAlaTrpVal

                      50                                    60
LysValValGluGluLysAlaPheSerProGluValIleProMetPheSerAlaLeuSer

                      70                                    80
GluGlyAlaThrProGlnAspLeuAsnThrMetLeuAsnThrValGlyGlyHisGlnAla

                      90                                   100
AlaMetGlnMetLeuLysGluThrIleAsnGluGluAlaAlaGluTrpAspArgValHis

                     110                                   120
ProValHisAlaGlyProIleAlaProGlyGlnMetArgGluProArgGlySerAspIle

                     130                                   140
AlaGlyThrThrSerThrLeuGlnGluGlnIleGlyTrpMetThrAsnAsnProProIle

                     150                                   160
ProValGlyGluIleTyrLysArgTrpIleIleLeuGlyLeuAsnLysIleValArgMet

                     170                                   180
TyrSerProThrSerIleLeuAspIleArgGlnGlyProLysGluProPheArgAspTyr

                     190                                   200
ValAspArgPheTyrLysThrLeuArgAlaGluGlnAlaSerGlnGluValLysAsnTrp

                     210                                   220
MetThrGluThrLeuLeuValGlnAsnAlaAsnProAspCysLysThrIleLeuLysAla

                     230                                   240
LeuGlyProAlaAlaThrLeuGluGluMetMetThrAlaCysGlnGlyValGlyGlyPro

                     250                                   260
AsnSerProArgGlnLeuLeuSerGlyIleValGlnGlnGlnAsnAsnLeuLeuArgAla
            gp41>

                     270                                   280
IleGluAlaGlnGlnHisLeuLeuGlnLeuThrValTrpGlyIleLysGlnLeuGlnAla


                     290                                   300
ArgIleLeuAlaValGluArgTyrLeuLysAspGlnGlnLeuLeuGlyIleTrpGlyCys

                     310                                   320
SerGlyLysLeuIleCysThrThrAlaValProTrpAsnAlaSerTrpSerAsnLysSer

                     330                                   340
LeuGluGlnIleTrpAsnAsnMetThrTrpMetGluTrpAspArgGluIleAsnAsnTyr

                     350                                   360
ThrSerLeuIleHisSerLeuIleGluGluSerGlnAsnGlnGlnGluLysAsnGluGln

                     370
GluLeuLeuGluLeuAspLysTrpAlaSerLeuTrpAsnTrpPheAsnGlyAspPro;
```

facultativement modifiée par une ou plusieurs substitutions, insertions et/ou délétions d'acides aminés et/ou par une extension à une extrémité ou aux deux, avec la restriction qu'une protéine présentant une telle séquence modifiée est susceptible de liaison avec tant anti-p24 qu'anti-gp41 et qu'il existe un degré d'homologie d'au moins 75% entre les séquences modifiée et non modifiée.

7. Procédé de préparation d'une protéine recombinante par :
   (i) transformation d'une cellule hôte avec un vecteur qui incorpore un gène codant la protéine et qui est capable, dans la cellule hôte, d'exprimer la protéine;
   (ii) culture de la cellule hôte transformée de façon que la protéine soit exprimée, et
   (iii) collecte de la protéine;
   caractérisé en ce que le gène code une protéine suivant la revendication 6.

8. Dosage de l'anticorps anti-p24 et/ou anti-gp481 de HIV, lequel dosage comprend la mise en contact d'un échantillon à examiner avec une protéine capable de se lier à l'anticorps anti-p24 et/ou anti-gp41 de HIV-1 et la détermination du fait que l'anticorps se lie ou non à la protéine, caractérisé en ce que la protéine est une protéine suivant la revendication 6.

9. Trousse d'essai à utiliser dans un dosage de l'anticorps anti-p24 et/ou anti-gp41 de HIV-1, laquelle trousse comprend une protéine capable de se lier à l'anticorps anti-p24 et/ou anti-gp41 de HIV-1 et des moyens pour déterminer si l'anticorps contenu dans un échantillon à examiner se lie ou non à la protéine, caractérisée en ce que la protéine est une protéine suivant la revendication 6.

10. Composition pharmaceutique comprenant un excipient ou diluant pharmaceutiquement acceptable et une protéine suivant la revendication 6, comme constituant actif.

pXY460 gp41

```
                                          30                                             60
ATG AAT TCC CCC AGA CAA TTA TTG TCT GGT ATA GTG CAG CAG CAG AAC AAT TTG CTG AGG
Met Asn Ser Pro Arg Gln Leu Leu Ser Gly Ile Val Gln Gln Gln Asn Asn Leu Leu Arg


                                          90                                            120
GCT ATT GAG GCG CAA CAG CAT CTG TTG CAA CTC ACA GTC TGG GGC ATC AAG CAG CTC CAG
Ala Ile Glu Ala Gln Gln His Leu Leu Gln Leu Thr Val Trp Gly Ile Lys Gln Leu Gln


                                         150                                            180
GCA AGA ATC CTG GCT GTG GAA AGA TAC CTA AAG GAT CAA CAG CTC CTG GGG ATT TGG GGT
Ala Arg Ile Leu Ala Val Glu Arg Tyr Leu Lys Asp Gln Gln Leu Leu Gly Ile Trp Gly


                                         210                                            240
TGC TCT GGA AAA CTC ATT TGC ACC ACT GCT GTG CCT TGG AAT GCT AGT TGG AGT AAT AAA
Cys Ser Gly Lys Leu Ile Cys Thr Thr Ala Val Pro Trp Asn Ala Ser Trp Ser Asn Lys


                                         270                                            300
TCT CTG GAA CAG ATT TGG AAT AAC ATG ACC TGG ATG GAG TGG GAC AGA GAA ATT AAC AAT
Ser Leu Glu Gln Ile Trp Asn Asn Met Thr Trp Met Glu Trp Asp Arg Glu Ile Asn Asn


                                         330                                            360
TAC ACA AGC TTA ATA CAC TCC TTA ATT GAA GAA TCG CAA AAC CAG CAA GAA AAG AAT GAA
Tyr Thr Ser Leu Ile His Ser Leu Ile Glu Glu Ser Gln Asn Gln Gln Glu Lys Asn Glu


                                         390                                            420
CAA GAA TTA TTG GAA TTA GAT AAA TGG GCA AGT TTG TGG AAT TGG TTT AAC GGG GAT CCC
Gln Glu Leu Leu Glu Leu Asp Lys Trp Ala Ser Leu Trp Asn Trp Phe Asn Gly Asp Pro
```

gp41 pXY460

FIGURE 1

pXY460 Gag

```
                                    30                                    60
ATG AAT TCC CCT GAC ACA GGA CAC AGC AGT CAG GTC AGC CAA AAT TAC CCT ATA GTG CAG
Met Asn Ser Pro Asp Thr Gly His Ser Ser Gln Val Ser Gln Asn Tyr Pro Ile Val Gln

                                    90                                   120
AAC ATC CAG GGG CAA ATG GTA CAT CAG GCC ATA TCA CCT AGA ACT TTA AAT GCA TGG GTA
Asn Ile Gln Gly Gln Met Val His Gln Ala Ile Ser Pro Arg Thr Leu Asn Ala Trp Val

                                   150                                   180
AAA GTA GTA GAA GAG AAG GCT TTC AGC CCA GAA GTG ATA CCC ATG TTT TCA GCA TTA TCA
Lys Val Val Glu Glu Lys Ala Phe Ser Pro Glu Val Ile Pro Met Phe Ser Ala Leu Ser

                                   210                                   240
GAA GGA GCC ACC CCA CAA GAT TTA AAC ACC ATG CTA AAC ACA GTG GGG GGA CAT CAA GCA
Glu Gly Ala Thr Pro Gln Asp Leu Asn Thr Met Leu Asn Thr Val Gly Gly His Gln Ala

                                   270                                   300
GCC ATG CAA ATG TTA AAA GAG ACC ATC AAT GAG GAA GCT GCA GAA TGG GAT AGA GTG CAT
Ala Met Gln Met Leu Lys Glu Thr Ile Asn Glu Glu Ala Ala Glu Trp Asp Arg Val His

                                   330                                   360
CCA GTG CAT GCA GGG CCT ATT GCA CCA GGC CAG ATG AGA GAA CCA AGG GGA AGT GAC ATA
Pro Val His Ala Gly Pro Ile Ala Pro Gly Gln Met Arg Glu Pro Arg Gly Ser Asp Ile

                                   390                                   420
GCA GAA ACT ACT AGT ACC CTT CAG GAA CAA ATA GGA TGG ATG ACA AAT AAT CCA CCT ATC
Ala Glu Thr Thr Ser Thr Leu Gln Glu Gln Ile Gly Trp Met Thr Asn Asn Pro Pro Ile

                                   450                                   480
CCA GTA GGA GAA ATT TAT AAA AGA TGG ATA ATC CTG GGA TTA AAT AAA ATA GTA AGA ATG
Pro Val Gly Glu Ile Tyr Lys Arg Trp Ile Ile Leu Gly Leu Asn Lys Ile Val Arg Met

                                   510                                   540
TAT AGC CCT ACC AGC ATT CTG GAC ATA AGA CAA GGA CCA AAA GAA CCT TTT AGA GAC TAT
Tyr Ser Pro Thr Ser Ile Leu Asp Ile Arg Gln Gly Pro Lys Glu Pro Phe Arg Asp Tyr

                                   570                                   600
GTA GAC CGG TTC TAT AAA ACT CTA AGA GCC GAG CAA GCT TCA CAG GAG GTA AAA AAT TGG
Val Asp Arg Phe Tyr Lys Thr Leu Arg Ala Glu Gln Ala Ser Gln Glu Val Lys Asn Trp

                                   630                                   660
ATG ACA GAA ACC TTG TTG GTC CAA AAT GCG AAC CCA GAT TGT AAG ACT ATT TTA AAA GCA
Met Thr Glu Thr Leu Leu Val Gln Asn Ala Asn Pro Asp Cys Lys Thr Ile Leu Lys Ala

                                   690                                   720
TTG GGA CCA GGA GCT ACA CTA GAA GAA ATG ATG ACA GCA TGT CAG GGA GTG GGA GGA CCC
Leu Gly Pro Ala Ala Thr Leu Glu Glu Met Met Thr Ala Cys Gln Gly Val Gly Gly Pro
```

pXY460

```
GGG GAT CCC
Gly Asp Pro
```

FIGURE 2

## FIGURE 3

```
        10         20         30         40         50         60
ATGAATTCCC CTGACACAGG ACACAGCAGT CAGGTCAGCC AAAATTACCC TATAGTGCAG
       >p18                                           >p24
        70         80         90        100        110        120
AACATCCAGG GGCAAATGGT ACATGAGGCC ATATCACCTA GAACTTTAAA TGCATGGGTA

       130        140        150        160        170        180
AAAGTAGTAG AAGAGAAGGC TTTCAGCCCA GAAGTGATAC CCATGTTTTC AGCATTATCA

       190        200        210        220        230        240
GAAGGAGCCA CCCCACAAGA TTTAAACACC ATGCTAAACA CAGTGGGGGG ACATCAAGCA

       250        260        270        280        290        300
GCCATGCAAA TGTTAAAAGA GACCATCAAT GAGGAAGCTG CAGAATGGGA TAGAGTGCAT

       310        320        330        340        350        360
CCAGTGCATG GAGGGCCTAT TGCACCAGGC CAGATGAGAG AACCAAGGGG AAGTGACATA

       370        380        390        400        410        420
GCAGGAACTA CTAGTACCCT TCAGGAACAA ATAGGATGGA TGACAAATAA TCCACCTATC

       430        440        450        460        470        480
CCAGTAGGAG AAATTTATAA AAGATGGATA ATCCTGGGAT TAAATAAAAT AGTAAGAATG

       490        500        510        520        530        540
TATAGCCCTA CCAGCATTCT GGACATAAGA CAAGGACCAA AAGAACCTTT TAGAGACTAT

       550        560        570        580        590        600
GTAGACCGGT TCTATAAAAC TCTAAGAGCC GAGCAAGCTT CACAGGAGGT AAAAAATTGG

       610        620        630        640        650        660
ATGACAGAAA CCTTGTTGGT CCAAAATGCG AACCCAGATT GTAAGACTAT TTTAAAAGCA

       670        680        690        700        710        720
TTGGGACCAG CAGCTACACT AGAAGAAATG ATGACAGCAT GTCAGGGAGT GGGAGGACCC

       730        740        750        760        770        780
AATTCCCCCA GACAATTATT GTCTGGTATA GTGCAGCAGC AGAACAATTT GCTGAGGGCT
       >gp41
       790        800        810        820        830        840
ATTGAGGCGC AACAGCATCT GTTGCAACTC ACAGTCTGGG GCATCAAGCA GCTCCAGGCA

       850        860        870        880        890        900
AGAATCCTGG CTGTGGAAAG ATACCTAAAG GATCAACAGC TCCTGGGGAT TTGGGGTTGC

       910        920        930        940        950        960
TCTGGAAAAC TCATTTGCAC CACTGCTGTG CCTTGGAATG CTAGTTGGAG TAATAAATCT

       970        980        990       1000       1010       1020
CTGGAACAGA TTTGGAATAA CATGACCTGG ATGGAGTGGG ACAGAGAAAT TAACAATTAC

      1030       1040       1050       1060       1070       1080
ACAAGCTTAA TACACTCCTT AATTGAAGAA TCGCAAAACC AGCAAGAAAA GAATGAACAA

      1090       1100       1110       1120       1130       1140
GAATTATTGG AATTAGATAA ATGGGCAAGT TTGTGGAATT GGTTTAACGG GGATCCCTAA
```

Fig. 4

Fig.5

Fig. 6

EP 0 307 149 B1

# Fig.7